# EUROPEAN PATENT SPECIFICATION

(11) **EP 1 935 357 B1**
(45) Date of publication and mention of the grant of the patent: **30.11.2011**
(21) Application number: 06820011.2
(22) Date of filing: 04.10.2006
(51) Int. Cl.: A61B 17/64

(54) **HINGE FOR RECIPROCALLY CONNECTING BARS AND/OR NEEDLES IN AN EXTERNAL FIXING DEVICE THAT IS USED TO REDUCE BONE FRACTURES**
GELENKSTÜCK ZUR WECHSELSEITIGEN VERBINDUNG VON STANGEN UND/ODER NADELN BEI EINER EXTERNEN FIXIERVORRICHTUNG ZUR REDUKTION VON KNOCHENBRÜCHEN
ARTICULATION DE SOLIDARISATION MUTUELLE DE BARRES ET/OU D'AIGUILLES DANS UN DISPOSITIF DE FIXATION EXTERNE POUR LA REDUCTION DE FRACTURES OSSEUSES

(30) Priority: 11.10.2005 ES 200502472
(43) Date of publication of application: 25.06.2008
(73) Proprietor: Implantvet S.L., 08310 Argentona (Barcelona) (ES)
(72) Inventor: TRILLA MUNTAÑOLA, Victor, E-08310 Argentona (Barcelona) (ES)
(74) Representative: Curell Aguilà, Marcelino
(86) International application number: PCT/ES2006/000550
(87) International publication number: WO 2007/045702

(56) References cited:
- EP-A1- 0 700 664
- EP-A1- 1 016 380
- WO-A1-01/54597
- WO-A1-03/065911
- DE-A1- 10 246 418
- ES-A6- 2 002 332
- ES-T3- 2 138 714
- US-A1- 2005 059 866
- US-B1- 6 409 729
- US-B2- 6 565 564
- US-B2- 6 702 814

## Description

### Field of the invention

The invention is contained in the field of the external fixation of bone fractures, where an external fixation device is used consisting of pins that are inserted into the bone, locking rods and joints that enable said rods to lock with said pins or for said rods to lock together.

In particular, the invention relates to an articulated joint for mutually locking said rods and/or pins, of the type that enables the rods or the pins to be fixed to the articulated joint by inserting them sideways under pressure so that they are held ready for the final tightening to provide an effective block. More particularly, the invention relates to an articulated joint of the type comprising a first support for rods and a second support for rods or pins, said supports being joined together by means of a rotary link around one single rotary axis, with the first support consisting of a pair of jaws which rest on one another via the action of elastic means and which together define at least one groove in which a rod is inserted sideways, against the action of said elastic means, with one rod being retained under pressure in said groove. The articulated joint also comprises tightening means that pass through said first and second supports along said rotary axis to tighten them against one another while also managing to block the relative angular position between said supports and tighten said jaws one against the other which consequently blocks said rod between said jaws.

### State of the art

Document EP0700664 describes an articulated joint of the type described at the beginning, wherein the second support is formed in the same way as the first, in other words it consists of a pair of jaws that together define a groove intended to receive a rod or pin in the sideways direction. A spring arranged between the two pairs of jaws keeps them separated, while maintaining the jaws in each pair in a tightened state. The groove defined in each pair of jaws is unique (in fact, this joint's particular design does not allow a larger number of grooves to be arranged in the jaws). Therefore, this articulated joint is not suitable for receiving more than two rods or pins. Moreover, it will be observed that the supports in this articulated joint are made up of four parts (the four jaws), and since these parts do not have a simple geometry, the joint's manufacturing costs are high.

Document US6565564 discloses an articulated joint that is similar to the type of articulated joint described at the beginning, but which differentiates from it in that the two supports are linked by a system that is articulated around two axes, thereby offering a greater degree of freedom in the fixation angle between the rods and pins. As in the previous case, the two supports each consist of a pair of jaws, but in this case, the pair of jaws forming the second support forms various parallel grooves, so that the articulated joint can receive several pins at the same time. However, this advantage is compensated negatively by the fact that the articulated joint consists of a larger number of parts, which are more complicated, and that the elastic means do not act upon the first support that receives the rods, thereby making it difficult to position said rods.

Document WO03065911 describes an articulated joint that also resembles the type of articulated joint described at the beginning, but which differentiates from it in that the two supports are linked by a ball articulated joint that allows any fixation angle between the rods and pins. As in the previous cases, the two supports each consist of a pair of jaws. Each jaw defines a single groove for receiving a rod or a pin, whereby this articulated joint essentially suffers from the same drawbacks as those cited with respect to Document EP0700664: complexity and manufacturing cost of the parts, and the fact that it is impossible to fix more than two rods or pins.

### Disclosure of the invention

The aim of the invention is to overcome the drawbacks of the state of the art by means of an articulated joint that has a particularly simple structure and which, in one particular embodiment of the invention, makes it possible to design said first and second supports so that each of them can receive two rods or two pins.

The articulated joint according to the invention is of the type indicated at the beginning, and it is characterized in that said second support consists of a single part that has a flat face that is maintained, by the action of said elastic means, resting against one corresponding flat face provided on a first of said jaws, and in that said flat face of the second support has at least one semi-groove along one edge of said second support, with said semi-groove defining, with said flat face of the first jaw, a channel into which a rod or a pin is inserted sideways, against the action of said elastic means, and retained under pressure in said channel.

It is worth highlighting that the articulated joint according to the invention does not consist of two pairs of jaws as in the state of the art, and that instead it has the particular feature whereby the set of the two supports consists of just three parts: on the one hand, the two jaws forming the first support that is intended to receive the rods and, on the other hand, the single part forming the second support that is intended to receive the pins. The basis of the operating principle of the second support is that each pin is fixed to the articulated joint thanks to the fact that it is housed in the semi-groove on the flat face of said second support and at the same time rests on the flat face of the first jaw. Fixing the pin according to this principle is efficient for any respective angular position between the supports, with one of the supports being able to rotate with respect to the other around the rotary axis without affecting the correct fixing of the pin, as said pin slides in contact with said flat face of the first jaw. This operating principle is robust and makes it possible to design particularly simple parts, which reduces the weight of the articulated joint and its manufacturing cost with respect to the state of the art joints.

Preferably, said jaws forming the first support have mutual engaging means and said jaws form two of said grooves arranged on one and the other side of said engaging means, so that the first support can hold up to two rods at the same time, with it being possible to insert and withdraw one of the rods without affecting the fixation of the other one. This particular feature is especially advantageous for fixing diaphysary (the ends of the bone are not affected) multi-fragmentary (having a large number of bone fragments) bone fractures, where the pins next to the focal point of the fracture are placed at a considerable distance from one another, which means the focal point of the fracture is unstable. In these situations, it is necessary to increase the stability of the fixation device in order to compensate the distance between the pins in the main fragments. This is achieved in a particularly effective manner by means of an articulated joint according to the invention, which makes it possible to place two parallel rods in the first support and one pin in the second support. With respect to the state of the art, this possibility offers easier assembly, considerably reduced unit weight and lower material cost. Also, in these multi-fragmentary fractures it is particularly advantageous to be able to withdraw one of the rods without having to dismantle the unit, as this means it is possible to increase the elasticity of the fixation in the final stage of the bone consolidation. This possibility of easily withdrawing one of the rods makes interventions easier in the final consolidation stage and leads to less time being needed for consolidation.

In an advantageous embodiment of the invention said two grooves have a different cross-sectional size. As each groove allows one rod to be fitted that has a diameter within a certain range (with the minimum diameter being approximately that of the groove and the maximum diameter being that permitted by the passage of the groove through which a rod is inserted under pressure against the action of the elastic means), as there are two different sized grooves, it is possible to cover a more extensive range of rod diameters. Consequently, a single articulated joint model is suitable for diverse applications that require different size rods.

Also, preferably, said second support has two of said semi-grooves along respective opposite edges of said second support, whereby the second support can hold up to two rods or two pins at the same time, with it being possible to insert and withdraw each rod or pin sideways, without affecting the fixation of the other one. This particular feature is especially advantageous for fixing the bone fractures that occur at the ends of long bones (in the metaphyseal area) and wherein the epiphysary fragment is small. In these situations, two pins have to be positioned in a very small space. However, the state of the art joints do not allow for this, as two joints have to be provided (one for each pin) and there is no physical space to house them. This problem is overcome by using an articulated joint according to the invention, wherein the second support can receive two pins.

In an advantageous embodiment, said two semi-grooves have a different cross-sectional size. By virtue of this arrangement, the second support can receive rods or pins with a wider diameter range, whereby one and the same articulated joint can be used for different applications that require different size rods or pins.

By combining these characteristics, a particularly advantageous embodiment of the invention is envisaged, whereby the first support has two grooves, preferably with different sections, and the second support has two semi-grooves, preferably with different sections. An articulated joint of this type according to the invention allows for multiple configurations, and it is possible to position none, one or two rods in the first support, and in the second support, also, none, one or two rods or pins. Moreover, an articulated joint of this type can receive very different size diameter pins and rods. By virtue of all this, a single articulated joint model can be applied to reduce different type bone fractures, and to a great variety of bones, including long bones, ranging from the longest such as the femur to the smallest such as the metacarpals, as well as flat bones such as the scapula or pelvis, both in different size humans and animals.

Preferably, said engaging means consist of a central projection and a corresponding central hollow each arranged in one of said jaws, with said central projection engaging in said central hollow with slight play which allows a slight sideways inclination relatively between said jaws. This arrangement is simple and efficiently guarantees the system for inserting the rods or pins sideways under pressure. It also acts efficiently when two grooves are provided in the first support and two semi-grooves in the second one, separated by said engaging means. Preferably, the surfaces of said flat face of the first jaw and of said flat face of the second support are rough, so as to more effectively block the relative rotation between the two supports when the unit is finally tightened using the tightening means.

Also, preferably, the surfaces of said grooves and of said semi-grooves are rough, so as to more effectively lock said rods and pins inserted into said grooves and channels when tightening the unit at the end with the tightening means.

Preferably, said tightening means consist of a dowel that passes through said jaws and said second support via corresponding holes aligned in said rotary axis, with said dowel having a seating head that rests on the second of said jaws and an opposite threaded end that threads into a corresponding female part, which preferably consists of the second support. The invention contemplates a preferable embodiment wherein said elastic means consist of a spring through which said dowel passes, with said spring resting with one end against said head of the dowel and with the other end against a seating surface arranged on said second jaw. These arrangements offer a simple and particularly efficient embodiment of the tightening means and the elastic means.

### Brief descridtion of the drawings

Other advantages and characteristics of the invention will be appreciated from the following description, in which, in a non-limiting manner, a preferred embodiment of the invention is described, with reference to the accompanying drawings, in which:
Fig. 1 shows an exploded perspective view of an articulated joint according to the invention;
Fig. 2 shows a sectional view of the articulated joint in Fig. 1, in the assembled position;
Figs. 3a, 3b, 3c, respectively, show upper, front and lower views of one of the jaws (the second one) forming the first support of the joint;
Figs. 4a, 4b, 4c, respectively, show upper, front and lower views of the other jaw (the first one) of said first support;
Figs. 5a, 5b, 5c, respectively, show upper, front and lower views of the single part forming the second support of the joint;
Fig. 6 shows a perspective view of the articulated joint in an assembly, which holds one rod and one pin;
Figs. 7 and 8 show diagrammatic views of two possible assemblies of an external fixation device for reducing bone fractures applying the articulated joint of the invention.

### Detailed description of an embodiment of the invention

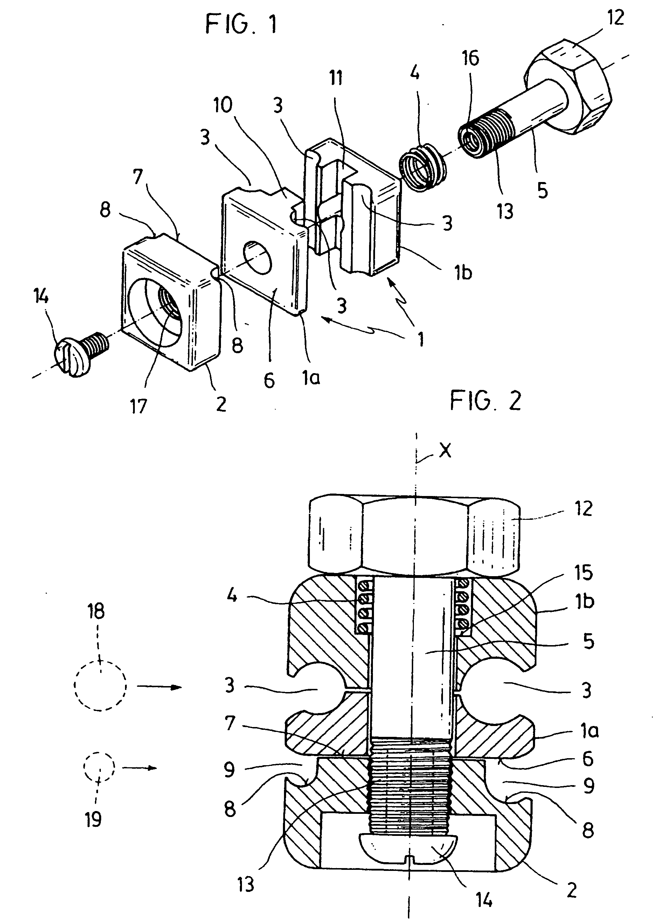

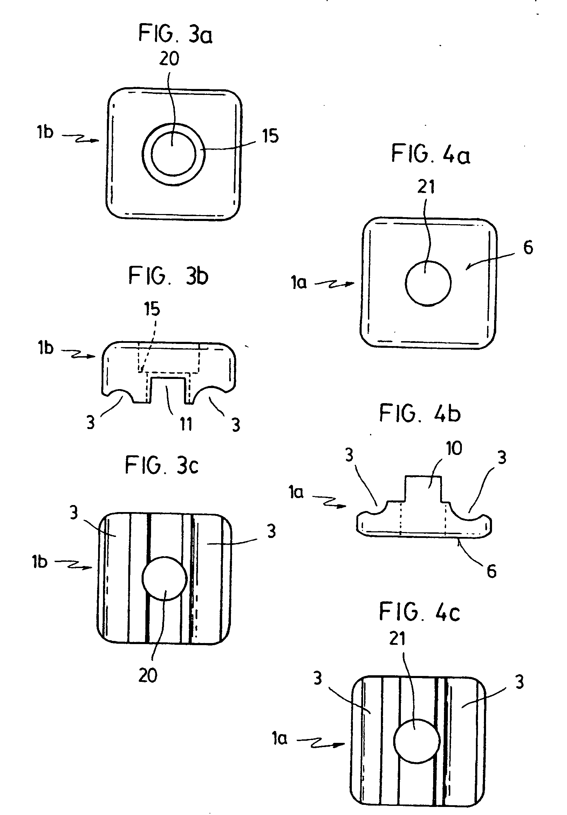

Figs. 1 and 2 show an embodiment of the articulated joint of the invention in an assembled position without any inserted pins or rods. The articulated joint consists of three basic parts: on the one hand, a pair of jaws 1a, 1 b that together form a first support 1 intended to receive some rods 18 in the sideways direction and, on the other hand, a single part 2 that forms a second support intended to receive both rods 18 and pins 19 in the sideways direction. These three parts are shown in Figs. 3, 4 and 5. Also, the articulated joint includes an assembled threaded dowel 5 that constitutes tightening means for said parts and a spring 4 that constitutes the elastic means to ensure that the rods and pins are fixed, as will be seen later. The two jaws 1a, 1b and part 2 each have a central through hole, respectively 21, 20 and 17, through which dowel 5 passes, centring the three parts on one single axis X.

The two jaws 1a, 1b mutually define two parallel grooves 3 that are open at respective parallel sides of the first support 1 (Fig. 2). Said grooves 3 receive rods 18 sideways under pressure and, thanks to the action of spring 4, they form a fixing mechanism for holding said rods. In order to insert a rod 18 sideways into one of grooves 3, said rod is placed parallel to groove 3 and a sideways pressure is applied, which, by working against the action of spring 4, causes jaws 1a, 1b to separate slightly, which allows rod 18 to be inserted in groove 3. Rod 18 thereby remains fixed in groove 3, resting under pressure on the convex surfaces of jaws 1a, 1b that together form groove 3. The pressure applied by spring 4 is high enough to prevent rod 18 from coming out of groove 3 sideways, but it does allow said rod to move in the axial direction providing that the unit has not been tightened by threaded dowel 5.

Jaws 1a, 1b also have mutual engaging means made up of a central projection 10 arranged on first jaw 1 a and a corresponding central hollow 11 arranged on second jaw 1b (an equivalent alternative solution would consist in arranging projection 10 on second jaw 1 b and hollow 11 on first jaw 1 a). Projection 10 and hollow 11 have a complementary parallelepiped shape and engage with slight play that allows a slight sideways inclination relatively between jaws 1a, 1b, which makes it easier to insert rods 18 sideways into grooves 3.

Part 2, for its part, has a flat face 7 on which it rests, by virtue of the action of spring 4, on a corresponding flat face 6 of first jaw 1a. Part 2 also has two parallel semi-grooves 8 along two edges of its flat face 7. In the assembled position of the articulated joint (Fig. 2), said semi-grooves 8, together with the flat face 6 of first jaw 1a, form open channels 9. Said channels 9 receive rods 18 or pins 19 sideways under pressure and, thanks to the action of spring 4, form a fixing mechanism for holding said rods or pins. In order to insert a rod 18 or pin 19 sideways into one of channels 9, said rod or pin is placed parallel to channel 9 and sideways pressure is applied which, working against the action of spring 4, causes part 2 to separate slightly with respect to jaw 1a which enables the rod or pin to be inserted into said channel. The rod or pin thereby remains fixed in channel 9, resting under pressure on one side on the convex surface of semi-groove 8 of part 2 and on the other side on flat face 6 of first jaw 1a. Here also, the pressure applied by spring 4 is high enough to prevent the rod or pin from coming out of channel 9 sideways, but it allows said rod or pin to move in the axial direction providing the unit has not been tightened by threaded dowel 5.

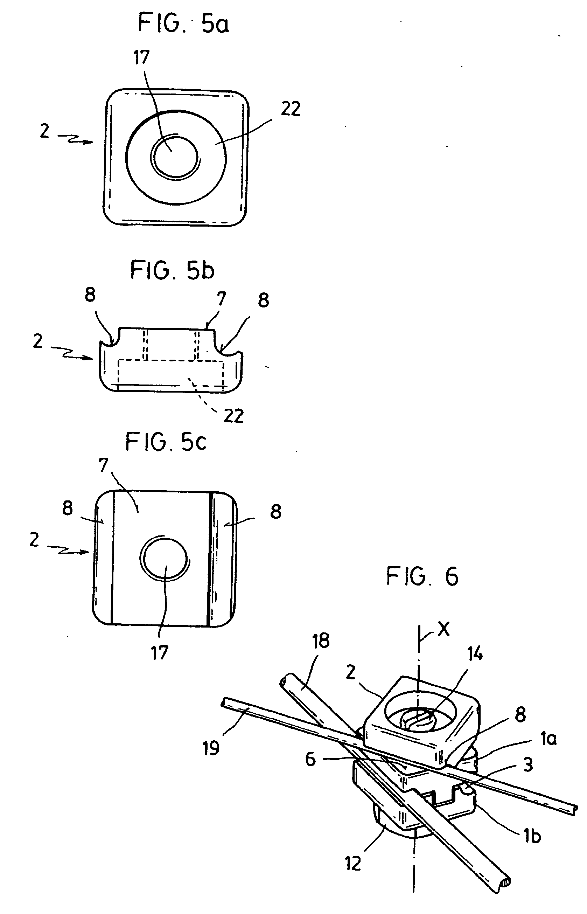

Fig. 6 shows an assembly of the articulated joint supporting a rod 18 and a pin 19. Providing the unit has not been tightened by threaded dowel 5, the first support that consists of jaws 1a, 1b, which is integral with rod 18, can rotate around axis X with respect to the second support that consists of single part 2, which is integral with pin 19, whereby a user can easily adjust the desired angle between rod 18 and pin 19. Said rotation around axis X is possible thanks to the fact that pin 19 slides on flat face 6 of first jaw 1 a.

Although in Fig. 6 a single rod 18 has been shown, fixed to the first support, and one single pin 19 fixed to the second support, it is perfectly possible to fix two rods to the first support and two pins (or alternatively two rods) to the second support, without thereby varying the operating principle of the joint.

As can be appreciated in Figs. 2 to 5, the two grooves 3 have a different size and the two semi-grooves 8 have also a different size, whereby one single articulated joint model is suitable for covering a wide range of rod and pin diameters.

The two jaws 1 a, 1b and single part 2 are made of stainless steel and they are produced by moulding, using a microcasting procedure. After moulding, the parts are polished to obtain a good surface finish, but the flat faces 6, 7 and the surfaces of grooves 3 and of semi-grooves 8 are left unmachined, so that they remain rough. When the unit is tightened by threaded dowel 5, this roughness is advantageous, because it helps to efficiently block part 2 with respect to first jaw 1a, as well as rods 18 and pins 19 inserted into grooves 3 and channels 9.

As can be seen in Figs. 1 and 2, dowel 5 passes through jaws 1a and 1 b via the respective holes thereof 21, 20 and with its threaded section 13 it threads through a threaded through hole 17 arranged in the centre of part 2. Dowel 5 has a hexagonal head 12 that rests on second jaw 1 b and the one that the user acts upon to tighten the unit. Spring 4 is coaxial with respect to dowel 5 and rests on one side on hexagonal head 12 and on the other side on a seating surface 15 of second jaw 1 b. The end of dowel 5 has a threaded hole 16 into which a screw 14 is threaded, which acts as an endstop to prevent a user from completely unthreading dowel 5 and accidentally causing the articulated joint to dismantle. The head of screw 14 is protected in a vacuum 22 made in the outer surface of part 2.

A fixation device for a bone fracture using an articulated joint according to the invention is basically assembled as follows. First of all, pins 19 are fixed to the bone. Then, the end of the pins are held in the joints, by inserting them sideways under pressure in channels 9, and the locking rods 18 are assembled, by inserting them sideways under pressure in grooves 3. Finally, the joints are tightened by fully threading dowel 5, thereby managing to block the relative rotation of part 2 with respect to the pair of jaws 1 and the axial sliding of the rods and pins.

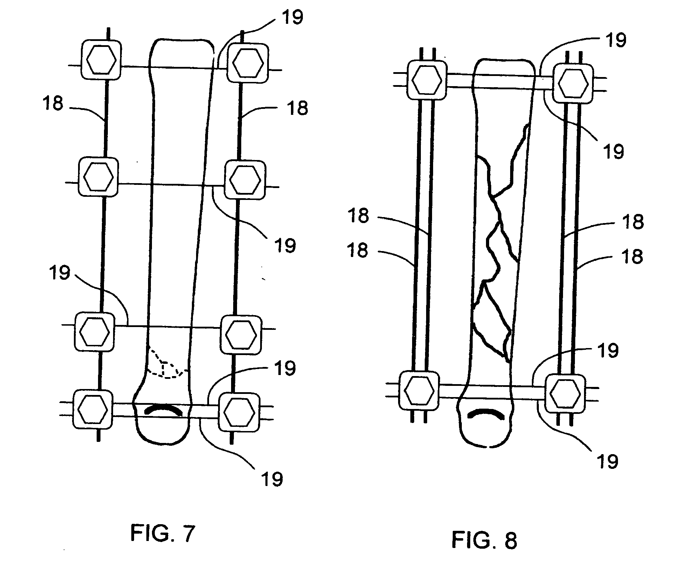

Figs. 7 and 8 diagrammatically show a particularly advantageous use of the joint. Fig. 7 shows the case where eight joints according to the invention are used to assemble an external fixation device of a fracture. By virtue of the articulated joint according to the invention, two parallel pins 19 are provided in a small space, fixing both of them to one and the same articulated joint (the two lower joints in the figure). Fig. 8 shows an assembly that uses four joints according to the invention. In this case, in addition to providing two parallel pins 19 fixed to one and the same joint, the rods 18 have been arranged parallel in twos, fixed in pairs to one and the same joint.

The person skilled in the art will understand that the embodiment described above is merely a non-limiting example, with respect to which various variations can be envisaged, without thereby departing from the scope of the invention. Thus, in particular, some joints can be envisaged in which a different number of grooves 3 and semi-grooves 8 are arranged, or in which grooves 3 and semi-grooves 8 have a section that is different to the one shown in the figures.

## Claims

1. Articulated joint for mutually locking rods and/or pins in an external fixation device for reduce bone fractures, with said articulated joint comprising a first support (1) for rods and a second support (2) for rod or pins, said supports (1, 2) being joined together by a rotary link around one single rotation axis (X), with said first support (1) consisting of a pair of jaws (1a, 1b) that rest against each other by virtue of the action of elastic means (4) and which define together at least one groove (3) in which a rod (18) is inserted sideways, against the action of said elastic jeans (4), and retained under pressure in said groove (3), with said articulated joint also comprising tightening means (5) that pass through said first (1) and second (2) supports along said rotation axis (X) to tighten them one against the other, at the same time blocking the relative angular position between said supports (1, 2) and tightening said jaws (1a, 1b) one against the other to consequently block said rod (18) between said jaws (1a, 1b), **characterized in that** said second support (2) consists of a single part that has a flat face (7) which rests, by virtue of the action of said elastic means (4), against a corresponding flat face (6) provided on a first (1a) of said jaws, and **in that** said flat face (7) of second support (2) has at least one semi-groove (8) along one edge of said second support (2), with said semi-groove (8) limiting together with said flat face (6) of first jaw (1a) a channel (9) into which a rod (18) or pin (19) is inserted sideways, against the action of said elastic means (4), and retained under pressure in said channel (9).

2. Articulated joint according to claim 1, **characterized in that** said jaws (1a, 1b) forming first support (1) have mutual engaging means (10, 11) and said jaws (1a, 1b) form two of said grooves arranged on one and the other side of said engaging means (10, 11).

3. Articulated joint according to claim 2, **characterized in that** said two grooves (3) have a different cross-sectional size.

4. Articulated joint according to claims 2 or 3, **characterized in that** said second support (2) has two of said semi-grooves (8), along respective opposite edges of said second support (2).

5. Articulated joint according to claim 4, **characterized in that** said two semi-grooves (8) have a different cross-sectional size.

6. Articulated joint according to any of the claims 2 through 5, **characterized in that** said engaging means consist of a central projection (10) and a corresponding central hollow (11) each arranged in one of said jaws (1a, 1b), with said central projection (10) engaging in said central hollow (11) with slight play that allows a slight lateral inclination relatively between said jaws (1a, 1b).

7. Articulated joint according to any of the claims 1 through 6, **characterized in that** the surfaces of said flat face (6) of the first jaw and said flat face (7) of the second support (2) are rough.

8. Articulated joint according to any of the claims 1 through 7, **characterized in that** the surfaces of said grooves (3) and said semi-grooves (8) are rough.

9. Articulated joint according to any of the claims 1 through 8, **characterized in that** said tightening means consist of a dowel (5) that passes through said jaws (1a, 1b) and said second support (2) via corresponding holes aligned in said rotary axis (X), with said dowel (5) having a seating head (12) that rests on the second (1b) of said jaws and an opposite threaded end (13) that threads into a corresponding female part (2).

10. Articulated joint according to claim 9, **characterized in that** said female part is the second support proper (2).

11. Articulated joint according to claims 9 or 10, **characterized in that** said elastic means consist of a spring (4) through which said dowel (5) passes, with said spring (4) resting with one end against said head (12) of the dowel (5) and with the other end against a seating surface (15) provided on said second jaw (1b).

## Patentansprüche

1. Gelenkstoß zum gegenseitigen Arretieren von Stangen und/oder Stiften in einer außenliegenden Fixierungsvorrichtung zum Richten von Knochenbrüchen, wobei der Gelenkstoß eine erste Halterung (1) für Stangen und eine zweite Halterung (2) für Stangen oder Stifte umfasst, wobei die Halterungen (1,2) durch eine drehbare Verbindung um eine einzige Rotationsachse (X) miteinander verbunden sind, wobei die erste Halterung (1) aus einem Paar Backen (1a, 1b) besteht, die aufgrund der Wirkung von elastischen Mitteln (4) aneinander liegen und die zusammen wenigstens eine Rinne (3) definieren, in die eine Stange (18) seitlich gegen die Wirkung der elastischen Mittel (4) eingesetzt ist, und unter Druck in der Rinne (3) gehalten wird, und wobei der Gelenkstoß weiterhin Befestigungsmittel (5) umfasst, die durch die erste (1) und zweite (2) Halterungen entlang der Rotationsachse (X) hindurchgehen, um sie gegeneinander festzuziehen, und gleichzeitig die relative Winkelposition zwischen den Halterungen (1,2) zu blockieren und die Backen (1a, 1b) gegeneinander festzuziehen, um somit die Stange (18) zwischen den Backen (1a, 1b) zu blockieren, **dadurch gekennzeichnet, dass** die zweite Halterung (2) aus einem einzigen Teil besteht, das eine plane Seite (7) aufweist, die aufgrund der Wirkung der elastischen Mittel (4) gegen eine korrespondierende plane Seite (6) anliegt, die an einer ersten (1a) der Backen bereitgestellt ist und **dadurch**, dass die plane Seite (7) der zweiten Halterung (2) wenigstens eine Halbrinne (8) entlang einer Kante der zweiten Halterung (2) besitzt, wobei die Halbrinne (8) zusammen mit der ersten planen Seite (6) der ersten Backe (1a) ein Kanal (9) begrenzt, in den eine Stange (18) oder Stift (19) seitlich gegen die Wirkung der elastischen Mittel (4) eingesetzt ist und unter Druck in dem Kanal (9) gehalten wird.

2. Gelenkstoß gemäß Anspruch 1, **dadurch gekennzeichnet, dass** die Backen (1a, 1b), die die erste Halterung (1) ausbilden, wechselseitige Ineingriffnahmemittel (10,11) aufweisen und die Backen (1a, 1b) zwei der Rinnen ausbilden, die an einer und der anderen Seite der Ineingriffnahmemittel (10,11) angeordnet sind.

3. Gelenkstoß gemäß Anspruch 2, **dadurch gekennzeichnet, dass** die zwei Rinnen (3) unterschiedliche Querschnitte aufweisen.

4. Gelenkstoß gemäß Anspruch 2 oder 3, **dadurch gekennzeichnet, dass** die zweite Halterung (2) zwei Halbrinnen (8) entlang entsprechenden gegenüberliegenden Kanten der zweiten Halterung (2) aufweist.

5. Gelenkstoß gemäß Anspruch 4, **dadurch gekennzeichnet, dass** die zwei Halbrinnen (8) unterschiedliche Querschnitte aufweisen.

6. Gelenkstoß gemäß einem der Ansprüche 2 bis 5, **dadurch gekennzeichnet, dass** die Ineingriffnahmemittel aus einer zentralen Ausbuchtung und einer korrespondierenden Vertiefung (11) bestehen, wobei jede in einer der Backen (1a, 1b) angeordnet ist, wobei die zentrale Ausbuchtung (10) mit geringem Spiel in die Vertiefung (1) eingreift, was eine leichte laterale Neigung im Verhältnis relativ zwischen den Backen (1a, 1b) erlaubt.

7. Gelenkstoß gemäß einem der Ansprüche 1 bis 6, **dadurch gekennzeichnet, dass** die Flächen der planen Seite (6) der ersten Backe und die plane Seite (7) der zweiten Halterung (2) rau sind.

8. Gelenkstoß gemäß einem der Ansprüche 1 bis 7, **dadurch gekennzeichnet, dass** die Flächen der Rinnen (3) und der Halbrinnen (8) rau sind.

9. Gelenkstoß gemäß einem der Ansprüche 1 bis 8, **dadurch gekennzeichnet, dass** die Befestigungsmittel aus einem Dübel (5) bestehen, der durch die Backen (1a, 1b) und die zweite Halterung (2) durch korrespondierende Löcher hindurchgeht, die in der Rotationsachse (X) ausgerichtet sind, wobei der Dübel (5) einen Aufsitzkopf (12), der an der zweiten (1b) der Backen anliegt und ein gegenüberliegendes Gewindeende (13) aufweist, das in ein korrespondierendes Mutterteil (2) eingeschraubt wird.

10. Gelenkstoß gemäß Anspruch 9, **dadurch gekennzeichnet, dass** das Mutterteil die passende zweite Halterung (2) ist.

11. Gelenkstoß gemäß Anspruch 9 oder 10, **dadurch gekennzeichnet, dass** die elastischen Mittel aus einer Feder (4) bestehen, durch die der Dübel (5) hindurchgeht, wobei die Feder (4) mit einem Ende an den Kopf (12) des Dübels (5) und mit dem anderen Ende gegen eine Aufsitzfläche (15) anliegt, die auf der zweiten Backe (1b) bereitgestellt ist.

## Revendications

1. Raccord articulé pour verrouiller mutuellement des tiges et/ou des broches dans un dispositif de fixation externe pour réduire des fractures osseuses, ledit raccord articulé comprenant un premier support (1) pour des tiges et un deuxième support (2) pour des tiges ou des broches, lesdits supports (1, 2) étant reliés conjointement par une liaison rotative autour d'un seul axe de rotation (X), ledit premier support (1) consistant en une paire de mâchoires (1a, 1b) qui reposent l'une contre l'autre grâce à l'action de moyens élastiques (4) et qui définissent conjointement au moins une gorge (3) dans laquelle une tige (18) est insérée latéralement, contre l'action desdits moyens élastiques (4), et maintenue sous pression dans ladite gorge (3), ledit raccord articulé comprenant également des moyens de serrage (5) qui passent à travers lesdits premier support (1) et deuxième support (2) le long dudit axe de rotation (X) pour les serrer l'un contre l'autre, en verrouillant en même temps la position angulaire rotative entre lesdits supports (1, 2) et en serrant lesdites mâchoires (1a, 1b) l'une contre l'autre pour verrouiller conséquemment ladite tige (18) entre lesdites mâchoires (1a, 1b), **caractérisé en ce que** ledit deuxième support (2) consiste en une seule partie qui possède une face plane (7) qui repose, grâce à l'action desdits moyens élastiques (4), contre une face plane correspondante (6) prévue sur une première (1a) desdites mâchoires, et **en ce que** ladite face plane (7) du deuxième support (2) possède au moins une demi-gorge (8) le long d'un bord dudit deuxième support (2), ladite demi-gorge (8) limitant conjointement avec ladite face plane (6) de la première mâchoire (1a) un canal (9) dans lequel une tige (18) ou une broche (19) est insérée latéralement, contre l'action desdits moyens élastiques (4), et maintenue sous pression dans ledit canal (9).

2. Raccord articulé selon la revendication 1, **caractérisé en ce que** lesdites mâchoires (1a, 1b) formant un premier support (1) possèdent des moyens de mise en prise mutuelle (10, 11) et lesdites mâchoires (1a, 1b) forment deux desdites gorges agencées sur l'un et l'autre côté desdits moyens de mise en prise (10, 11).

3. Raccord articulé selon la revendication 2, **caractérisé en ce que** lesdites deux gorges (3) possèdent une taille de section transversale différente.

4. Raccord articulé selon les revendications 2 ou 3, **caractérisé en ce que** ledit deuxième support (2) possède deux desdites demi-gorges (8), le long de bords opposés respectifs dudit deuxième support (2).

5. Raccord articulé selon la revendication 4, **caractérisé en ce que** lesdites deux demi-gorges (8) possèdent une taille de section transversale différente.

6. Raccord articulé selon l'une quelconque des revendications 2 à 5, **caractérisé en ce que** lesdits moyens de mise en prise consistent en une saillie centrale (10) et un évidement central correspondant (11) agencés chacun dans une desdites mâchoires (1a, 1b), ladite saillie centrale (10) étant en prise dans ledit évidement central (11) avec un léger jeu qui permet une légère inclinaison latérale relative entre lesdites mâchoires (1a, 1b).

7. Raccord articulé selon l'une quelconque des revendications 1 à 6, **caractérisé en ce que** les surfaces de ladite face plane (6) de la première mâchoire et ladite face plane (7) du deuxième support (2) sont rugueuses.

8. Raccord articulé selon l'une quelconque des revendications 1 à 7, **caractérisé en ce que** les surfaces desdites gorges (3) et desdites demi-gorges (8) sont rugueuses.

9. Raccord articulé selon l'une quelconque des revendications 1 à 8, **caractérisé en ce que** lesdits moyens de serrage consistent en un goujon (5) qui passe à travers lesdites mâchoires (1a, 1b) et ledit deuxième support (2) par des trous correspondants alignés dans ledit axe rotatif (X), ledit goujon (5) ayant une tête d'appui (12) qui repose sur la deuxième (1b) desdites mâchoires et une extrémité filetée opposée (13) qui s'ajuste par filetage dans une partie femelle correspondante (2).

10. Raccord articulé selon la revendication 9, **caractérisé en ce que** ladite partie femelle est proprement dit le deuxième support (2).

11. Raccord articulé selon les revendications 9 ou 10, **caractérisé en ce que** lesdits moyens élastiques consistent en un ressort (4) à travers lequel passe ledit goujon (5), ledit ressort (4) reposant avec une extrémité contre ladite tête (12) du goujon (5) et avec l'autre extrémité contre une surface d'appui (15) prévue sur ladite deuxième mâchoire (1b).
